# EUROPEAN PATENT SPECIFICATION

(11) **EP 1 684 719 B1**
(45) Date of publication and mention of the grant of the patent: **26.10.2011**
(21) Application number: 04811377.3
(22) Date of filing: 11.11.2004
(51) Int. Cl.: A61K 9/12, A61P 11/00

(54) **ALPHA 1-ANTITRYPSIN COMPOSITIONS AND TREATMENT METHODS USING SUCH COMPOSITIONS**
ALPHA1-ANTITRYPSIN-ZUSAMMENSETZUNGEN UND BEHANDLUNGSVERFAHREN UNTER VERWENDUNG DIESER ZUSAMMENSETZUNGEN
COMPOSITIONS D'ALPHA 1-ANTITRYPSINE ET PROCEDES DE TRAITEMENT METTANT EN OEUVRE DE TELLES COMPOSITIONS

(30) Priority: 14.11.2003 US 520549 P
(43) Date of publication of application: 02.08.2006
(73) Proprietor: Baxter International Inc., Deerfield, IL 60015 (US); Baxter Healthcare SA, 8304 Zurich (CH); Arriva Pharmaceuticals, Inc., Alameda, CA 94501 (US)
(72) Inventor: DURRANI, Manzer J., Plantation, FL 33322 (US); KUMAR, Harish, Fullerton, CA 92833 (US); KREIGER, Timothy, Lowell, MA 01854 (US); KABINGUE, Ken, Los Angeles, CA 90041 (US); MOSHER, Virginia, Lebanon, MO 65536 (US); BARR, Philip J., Oakland, CA 94619 (US); BATHURST, Ian C., Alameda, CA 94501 (US)
(74) Representative: Roques, Sarah Elizabeth
(86) International application number: PCT/US2004/038650
(87) International publication number: WO 2005/048985

(56) References cited:
- EP-A- 0 289 336
- US-A- 5 093 316
- US-A- 5 166 134
- US-A1- 2001 006 939

## Description

Alpha 1- Antitrypsin (AAT) is a protease inhibitor with relatively broad substrate specificity; its primary function is to inhibit elastase, but it is also an inhibitor of cathepsin G and proteinase 3. In addition to its activity as an inhibitor of these enzymes, AAT has also been shown to inhibit degranulation of lung mast cells, inhibit histamine release factors, inhibit the release of tumor necrosis factor (TNF) and inhibit the release of leukotriene B₄ from alveolar macrophages and cells.

AAT is synthesized primarily in the liver, but is also synthesized to a lesser extent in other cells, Including macrophages, intestinal epithelial cells and intestinal Paneth cells. In the liver, AAT is initially synthesized as a 52 kD precursor protein that subsequently undergoes post translational glycosylation at three asparagine residues, as well as tyrosine sulfonation. The resulting protein is secreted as a 55 kD native single-chain glycoprotein. The normal allotype of AAT is referred to as type M AAT (often referred to as protease inhibitor type M, or simply PiM). Once secreted into the plasma, the half-life of PiM is approximately 5 days.

Certain individuals have a genetic disorder that results in an AAT deficiency. These individuals are at increased risk for liver disease and/or pulmonary emphysema. The risk of pulmonary emphysema is increased because lung tissue in mammals is especially susceptible to the action of elastase, which, if uncontrolled, can degrade all major protein components of the alveolar interstitium (see, e.g., Smith, et al. (1989) J. Clin. Invest. 84:1145-1154).

AAT-deficiency is most frequently transmitted as an autosomal recessive trait that affects about 1 in 1700 individuals in most North American and Northern European populations. This particular form of AAT-deficiency is commonly referred to as protease inhibitor type Z (or simply PiZ). The mutation associated with PiZ is a single nucleotide substitution that causes a substitution of glutamate 342 with lysine. The evidence to date indicates that this substitution prevents normal protein folding of AAT. This mutation specifically appears to reduce the stability of the monomeric form of the protein and increase the formation of polymeric forms (see, e.g., Lomas et al. (1992) Nature 357:605-607; see also, Gadek, et al., (1982) "Alpha-1-Antitrypsin Deficiency", in The Metabolic Basis of Inherited Diesease (Stanbury, J.B., et al., Eds) McGraw-Hill, New York, pp. 1450-1467; and Carroll, et al. (1982) Nature 2988:329-334).

In the case of liver disease associated with AAT-deficiency, research indicates that the mutant PiZ form of AAT is retained as globules in the endoplasmic reticulum (ER) of hepatic cells. The retention of mutant AAT appears to be occasioned by the abnormal folding of PiZ form of the enzyme, which inhibits its transport from the ER to the Golgi (see, e.g., Carlson et al., (1988) J. Clin. Invest. 83:1183-90; Wu et al. (1994) Proc. Natl. Acad. Sci. 91:9014-9018; and Dycaico, et al. (1988) Science 242:1409-1412).

As noted above, a deficiency in AAT plays a central role in the pathogenesis of emphysema because of the key role that AAT plays in regulating elastase, cathepsin G and proteinase 3. Research, for instance, indicates that AAT accounts for more than 90% of neutrophil elastase inhibitor activity in pulmonary alveolar lavage fluid, suggesting that the destruction in lung tissue observed in individuals with AAT-deficiency is due to an imbalance in elastase and AAT within the lungs. The uncontrolled action of elastase, together with that of cathepsin G and proteinase 3, results in the slow but steady destruction of lung connective tissue and elasticity associated with the onset of pulmonary emphysema. There is some evidence indicating that environmental pollutants and cigarette smoke can aggravate the problem of AAT-deficiency by oxidizing what little AAT is present into an inactive form (see, e. g. , Hunnighake, et al. (1983) Am. Rev. Respir. Dis. 128: 833-838).

Administering AAT to individuals having AAT-deficiency has been the primary treatment mode to date. Often such treatment protocols involve administering AAT intravenously. There are at least two problems with this approach. The first problem is that large amounts of AAT must be administered intravenously to reach therapeutic levels in the lung. Second, while the use of recombinant AAT is advantageous because it can be readily obtained in relatively large quantities, this form of AAT has a short half-life in the circulation because recombinant AAT is less stable than the naturally occurring form.

There thus remains a need for new compositions for providing AAT in a stable form to patients, including recombinant forms. US 5,166,134 provides a method for the prophylaxis or direct treatment of allergic rhinitis which comprises nasally administering to a patient an aqueous solution of an effective amount of a serine protease inhibitor. US 2001/0006939 A1 relates to formulations of secretory leukocyte protease inhibitor (SLPI) for pulmonary delivery.

### BRIEF SUMMARY OF THE INVENTION

A variety of pharmaceutical compositions containing AAT are provided. The compositions are useful in treating a variety of diseases associated with AAT deficiency. The compositions generally include AAT, a stabilizing carbohydrate, a surfactant and an antioxidant. There is provided a pharmaceutical composition formulated as a powder, comprising an alpha 1-antitrypsin (AAT), trehalose, a surfactant and an antioxidant, wherein the AAT is:
(a) a native AAT;
(b) a recombinant AAT; or
(c) an AAT variant that has at least 85% amino acid sequence identity to SEQ ID NO: 1 and can inhibit elastase. There is also provided a composition according to the invention for use in a method for the treatment of a pulmonary disease associated with alpha 1-antitrypsin (AAT) deficiency, wherein the composition is to be administered to the lungs of a patient.

The compositions are formulated for administration to a patient via inhalation therapy. The compositions are formulated as powders and are administered by converting the powder to an aerosol. The AAT in the pharmaceutical composition can be of a variety of different forms including a naturally occurring AAT, a recombinant AAT, and a transgenic AAT. The AAT can be glycosylated or unglycosylated.

A number of different antioxidants can be used in the compositions.
Exemplary antioxidants include glutathione and methionine.

The solution based pharmaceutical compositions discussed herein usually include 1-100mg/ml AAT, 1-5% (w/v) trehalose, 0.01-0. 5%(w/v) surfactant, and 1-10 mM antioxidant. The AAT concentration in some compositions is somewhat lower at 10-50 mg/ml. Hence, in compositions formulated as powders, the AAT, trehalose, surfactant and antioxidant are present in amounts such that if the powder is solubilized in aqueous solution for administration to a patient the AAT concentration Is 1 -100 mg/ml, the trehalose concentration is 1-5% (w/v), the surfactant concentration is 0.01-0.5% (w/v), and the antioxidant concentration is 1-10 mM.

Certain other pharmaceutical compositions that are disclosed herein, comprise a recombinant alpha 1-antitrypsin (AAT), trehalose, a surfactant and an antioxidant, wherein the AAT/carbohydrate ratio (weight:weight) is 1:1 to 5:1.

A specific example of one formulation illustrating the relative amounts of the various components of the formulation is a 2 ml formulation containing 50 mg AAT/ml, 25 mg trehalose/ml, 5 mM methionine, and 0.02°C polysorbate 80. If lyophilized, such a composition yields 100 mg AAT, 50 mg trehalose, 1.5 mg methionine and 0.4 mg polysorbate 80. Another base formulation comprises 5% AAT, 2.5% carbohydrate (e.g., trehalose), 0.1-0.2% surfactant (e.g., Tween-80), 5 mM antioxidant (e.g., methionine) nd 10 mM buffer (e.g., sodium phosphate, at pH 7.4), all percentages being (w/v).

Pharmaceutical compositions of the foregoing compositions can be used In treating a variety of diseases that are correlated with AAT deficiency. For instance, these compositions can be used for treating a pulmonary disease associated with AAT deficiency. So, for example, certain methods Involve administering to the lungs of a patient a pharmaceutical composition that comprises an effective amount of AAT, trehalose, a surfactant and an antioxidant. The specific composition of these compositions are as Just described. As indicated above, the compositions are generally administered by inhalation. If the composition is a solid, the method involves converting the solid into an aerosol which the patient can inhale.

The Compositions can be used to treat a variety of diseases. Examples of diseases that can be treated by administering the disclosed compositions include a pulmonary disease associated with the activity of elastase, cathepsin G and/or proteinase 3. Certain treatments are effective in treating a pulmonary inflammatory disease associated with activation of neutrophiis, mast cells or T-celis. Other diseases that can be treated with some compositions include adult respiratory distress syndrome, neonatal respiratory distress syndrome and sepsis syndrome. Other specific examples of disease that can be treated include emphysema and asthma.

In some treatment methods, the patient is susceptible to the disease and the pharmaceutical composition is administered in a prophylactically effective amount. Whereas, in other instances, the patient has the disease and the pharmaceutical composition is administered in a therapeutically effective amount.

Still other methods for treating a pulmonary disease associated with AAT deficiency involve administering to the lungs of a patient a pharmaceutical composition that comprises unglycosylated recombinant AAT, trehalose and at least one additional stabilizing agent selected from the group consisting of a surfactant and an antioxidant, wherein the AAT/carbohydrate ratio is 1:1 to 5:1. Methods that Involve administering such compositions can be used to treat the foregoing diseases.

### DETAILED DESCRIPTION

### I. Definitions

Unless defined otherwise, all technical and scientific terms used herein have the meaning commonly understood by a person skilled in the art to which this invention belongs. The following references provide one of skill with a general definition of many of the terms used in this invention: Singleton et al., DICTIONARY OF MICROBIOLOGY AND MOLECULAR BIOLOGY (2d ed. 1994); THE CAMBRIDGE DICTIONARY OF SCIENCE AND TECHNOLOGY (Walker ed., 1988); THE GLOSSARY OF GENETICS, 5TH ED., R. Rleger et al. (eds.), Springer Verlag (1991): and Hale & Marham, THE HARPER COLLINS DICTIONARY OF BIOLOGY (1991). As used herein, the following terms have the meanings ascribed to them unless specified otherwise.

The term "AAT" as used herein generally refers to a protein having a native AAT amino acid sequence, as well as variants, fragments and modified forms regardless of origin or mode of preparation. The term thus encompasses both naturally occurring and recombinant forms of AAT, as well as glycosylated and unglycosylated forms. The term includes AAT isolated from natural sources (e.g., plasma), AAT produced by synthetic methods that are well known In the art, and/or by recombinant or transgenic means. "Naturally occurring AAT" or simply "native AAT" refers to AAT forms that can be isolated from natural sources (e.g., serum). The term naturally occurring AAT specifically encompass naturally occurring truncated or soluble forms, naturally occurring variant forms (e.g., alternatively spliced forms), naturally occurring allelic variants of AAT and forms including postranslational modifications (e.g., the glycosylation and sulfonation discussed in the Background). Specific examples of native AAT sequences are provided In U.S. Patent Nos. 4,599,311 and 4,711,848. An exemplary native AAT sequence is provided as SEQ ID NO:1 (see, also GenBank accession no. AAB59375). "Recombinant AAT" (rAAT) refers to AAT produced using genetic engineering, recombinant or transgenic techniques. Exemplary methods for producing recombinant AAT are discussed in U.S. Patent Nos. 4,599,311; 4,931,373; and 5,218,091. Recombinant AAT forms can be glycosylated or unglycosylated depending upon the organism in which the enzyme Is expressed. The term AAT can include both human forms of AAT, as well as other mammalian AATs (e.g., those from primates such as monkeys, chimpanzee and gorilla, as well as non-primates such as mice, rabbits, cows and swine).

"AAT variants" refer to proteins that are functional equivalents to a native sequence AAT protein and that have similar amino acid sequences and retain, to some extent, one or more of the activities of naturally occurring AAT. AAT activities include, but are not limited to, capacity to inhibit elastase, cathepsin G and/or proteinase 3. Other exemplary AAT activities include capacity to inhibit degranulation of lung mast cells, to inhibit histamine release factors, to inhibit the release of tumor necrosis factor (TNF) and/or to inhibit the release of leukotriene B₄ from alveolar macrophages and cells.

Variants also include fragments that retain AAT activity. Fragments include the active site of AAT and typically include at least 5-20 flanking amino acids on either side of the active site. Fragments usually include at least 25, 50, 75, 100, 150, 200, 250, 300 or 350 amino acids.

AAT variants also include proteins that are substantially identical to a native sequence of AAT. Such variants include proteins having amino acid alterations such as deletions, insertions and/or substitutions. A "deletion" refers to the absence of one or more amino acid residues In the related protein. The term "Insertion" refers to the addition of one or more amino acids In the related protein. A "substitution" refers to the replacement of one or more amino acid residues by another amino acid residue in the polypeptide. Typically, such alterations are conservative in nature such that the activity of the variant protein is substantially similar to a native sequence for AAT (see, e.g., Creighton (1984) Proteins, W.H. Freeman and Company). In the case of substitutions, the amino acid replacing another amino acid usually has similar structural and/or chemical properties, insertions and deletions are typically In the range of 1 to 5 amino acids, although depending upon the location of the insertion, more amino acids can be Inserted or removed. The variations can be made using methods known In the art such as site-directed mutagenesis (Carter, et al. (1986) Nud. Acids Res. 13:4331: Zoller et al. (1987) Nucl. Acids Res. 10:6487), cassette mutagenesis (Wells et al. (1985) Gene 34:315), restriction selection mutagenesis (Wells, et al. (1986) Philos. Trans. R. Soc. London SerA 317:415), and PCR mutagenesis (Sambrook, et al. (1989) Molecular Cloning, Cold Spring Harbor Laboratory Press).

AAT variants also include modified or derivative forms of AAT. Modified AAT generally refers to proteins In which one or more amino acids of a native sequence AAT have been altered to a non-naturally occurring amino acid residue. Such modifications can occur during or after translation and include, but are not limited to, phosphorylation, glycosylation, sulfonation, cross-linking, acylation and proteolytic cleavage.

Specific examples of variant forms of AAT are discussed, for example, In U.S. Patent Nos. 4,732,973 and 5,134,119, both of which are incorporated herein by reference in their entirety for all purposes. Exemplary variants are those in which the active site methionine is replaced with amino acids that are less susceptible to oxidation (e.g., valine, alanine, leucine, isoleucine, serine or threonine). Other specific variants and methods for producing variants generally are described in U.S. Patent No. 4,711,848.

The terms "identical" or percent "identity,' in the context of two or more nucleic acids or polypeptides, refer to two or more sequences or subsequences that are the same or have a specified percentage of nucleotide or amino acid residues that are the same, when compared and a!igned for maximum correspondence, as measured using a sequence comparison algorithm such as those described below for example, or by visual inspection.

The phrase "substantially identical," or other related phrases when used in the context of two nucleic acids or polypeptides, refers to two or more sequences or subsequences that have at least 80%, preferably at least 85%, more preferably at least 90%, 95% or 99% nucleotide or amino acid residue identity, when compared and aligned for maximum correspondence, as measured using a sequence comparison algorithm such as those described below for example, or by visual inspection. Preferably, the substantial identity exists over a region of the sequences that is at least about 40-60 residues in length, preferably over a longer region than 60-80 amino acids, more preferably at least about 90-100 residues, and most preferably the sequences are substantially identical over the full length of the sequences being compared, such as the coding region of a nucleotide for example.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, *e.g.*, by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection [*see generally*, Current Protocols in Molecular Biology, (Ausubel, F.M. et al., eds.) John Wiley & Sons, Inc., New York (1987-1999, including supplements such as supplement 46 (April 1999)]. Use of these programs to conduct sequence comparisons are typically conducted using the default parameters specific for each program.

### II. Overview

Compositions that are useful for stabilizing various proteins as a pharmaceutical formulation are provided, as well as methods of using such formulations in a variety of therapeutic and prophylactic treatment methods. One group of formulations that have been developed contain AAT. These formulations are useful in treating a variety of symptoms associated with AAT deficiency. "AAT deficiency" as used herein generally means that the endogenous AAT levels of an individual are insufficient to protect against the destructive activity of elastase or other proteases such as cathepsin G and proteinase 3. AAT levels can be insufficient because the AAT levels are lower than typical for a population of individuals and/or because elastase levels are elevated relative to the general population.

The formulations have been designed to address challenges common to protein formulations generally, as well as specific challenges associated with the delivery of AAT as a therapeutic agent. For instance, proteins for therapeutic use are often inherently unstable outside their natural biological environment. Common stability problems include non-covalent and covalent aggregation, deamidation, formation of cyclic imides, cleavage and oxidation. AAT, for example, is susceptible to oxidation because of the presence of an active site methionine and to aggregation. The formulations that are disclosed herein improve the stability of AAT relative to aqueous solutions of AAT that lack stabilizing agents, including the unglycosylated recombinant forms that are less stable than naturally occurring forms. The components of the formulations have also been selected so that the formulation can be readily nebulized to enhance delivery to the lungs or converted into an aerosol from dry powder. The formulations can be prepared In aqueous form, or lyophilized as a powder to further enhance stability. Consequently, the formulation can be administered by nebulizing liquid formulations or, in some instances, by converting the powder to a dry aerosol that can be inhaled.

### III. Compositions

### A. General

The formulations or compositions that are provided: 1) include components that stabilize proteins, 2) are In a form that can be readily converted into an aerosol, and 3) are compatible with lung tissue. Although much of the discussion herein focuses on formulations that contain AAT, those of ordinary skill in the art will recognize that the components of the disclosed formulations can be used to prepare formulations containing other proteins.

Components included in the compositions generally include one or more of the following: a simple sugar (e.g., a monosaccharide, a disaccharide or a trisaccharide), a surfactant/detergent, an antioxidant, and a buffering agent. Various other active ingredients can also optionally be included, such as one or more additional protease inhibitors and/or an anti-inflammatory agent.

The formulations can be prepared in various ways, Including: 1) as a liquid that can be subsequently nebulized, 2) as a powder that can be delivered by dry powder aerolization, or 3) as a liquid that Is lyophilized to a powder for storage, which in tum is later reconstituted as a liquid shortly before being administered to a patient. The concentrations of the components listed below generally assume a liquid formulation. The components in the powder formulations are present in appropriate amounts such that if the powder was reconstituted it would yield a liquid with the concentrations described herein.

### B. Protein

The AAT included in the formulation is defined above and can be naturally occurring, prepared by recombinant techniques and/or prepared by chemical synthesis. AAT from human plasma is commercial available as a parenteral product, PROLASTIN, produced by Bayer. Glycosylated AAT or unglycosylated AAT (see, e.g., U.S. Patent Nos. 4.599,311; 4,931.373; and 5.21.8.091) can be used in the formulations. Some formulations include an AAT fragment or variant as defined above. The sequences of such variants typically have an amino acid sequence that is substantially identical to an amino acid sequence of a naturally occurring AAT (see, e.g., U.S. Patent Nos. 4,711,848 and U.S. 4,599,311) and have one or more activities of AAT with a native sequence. So, for example, certain compositions include an AAT variant that: 1) has at least 85, 90 or 95% sequence identity to the native AAT sequence listed In SEQ ID NO:1, and 2) can inhibit elastase. Suitable variants that can be included in certain compositions include, for example, those discussed in U.S. Patent Nos. 4,732,973; 5,134,119; and U.S. Patent No. 4,711,848. The AAT can be a human or other mammalian AAT.

Salts and derivatives of the protein can be utilized as well. These can be prepared by established techniques commonly used with other proteins without adversely affecting the activity of the protein. Examples of suitable salts and derivatives that can be utilized In certain formulations include, but are not limited to, alkali metal salts, acid-addition salts and esters.

The protein typically has a purity of at least 90%, in other instances at least 95%, and in still other instances at least 98 or 99%. These purity values reflect the amount of AAT relative to other protein in the composition on a weight basis. The AAT concentration can vary, but in liquid formulations generally ranges from 1 -100 mg/ml, including any range therebetween. In other instances, the concentration is 1-50 mg/ml, and in still other instances 60-65 mg/ml. The AAT concentration in some formulations is less than 100, 75, 50, 25, 10 or 5 mg/ml. Expressed on a weight percentage basis, the AAT concentration typically ranges from 2-20% (w/v), and in some instances ranges from 5-15% (w/v). So, for example, some formulations have an AAT concentration of 5, 7, 9, 11, 13 or 15% (w/v).

### C. Carbohydrate

The compositions contain a simple carbohydrate (trehalose ). The carbohydrate functions, among other things, as an amorphous cryoprotectant and lyoprotectant, thus facilitating room temperature storage. The carbohydrate is selected to be compatible with lung tissue and, for formulations that are to be nebulized, compatible with nebulization. The results described infra indicate that trehalose satisfies these criteria well.

The concentration of the carbohydrate (trehalose) in a liquid formulation generally ranges from about 1 mg/ml to about 50 mg/ml, and in other instances about 10 mg/ml to about 50 mg/ml. Carbohydrate concentrations significantly above this level can result in too much carbohydrate being delivered during inhalation, which can result in the lung potentially becoming coated with carbohydrate. Thus, various formulations contain at least 1, 5, 10, 15, 20, 25, 30, 35, 40 or 45 mg/ml carbohydrate, but generally less than about 50 mg/ml. Expressed on a percentage basis, the carbohydrate concentration usually is up to about 5% (w/v). Thus, the carbohydrate concentration on weight percentage basis is usually.at least 1, 2, 3 or 4% but less than 5%. The protein/carbohydrate ratio (weight:weight) is usually adjusted so this ratio Is between about 1:1 - 5:1. Exemplary formulations (e.g., AAT formulations) thus have protein/carbohydrate ratios (w:w) of 1:1, 2:1, 3:1, 4:1 or 5:1.

### D. Surfactant

A surfactant that can be nebulized or converted into an aerosol and that is compatible with lung tissue is also typically incorporated into the protein formulations to enhance the stability of the protein. As used herein, the term "surfactant" has its usual meaning in the art and generally means a surface-active agent. Surfactants are also sometimes referred to in the art as wetting agents, surface tension depressants, detergents, dispersing agents or emulsifiers. The surfactant is useful in preventing aggregation of the protein in the formulation and in minimizing surface denaturation that can occur during filtration, freeze-thawing, freeze-drying and storage. Protecting against aggregation is important with formulations containing AAT, for instance, because AAT aggregates are less stable than the monomeric forms.

The surfactant can be selected from any surfactant or detergent that has the characteristics just listed. Typically, a non-ionic surfactant is utilized. Specific examples of suitable surfactants include, but are not limited to, various Polysorbate (USP/NF name) stabilizers (e.g., Polysorbate 80). Other suitable surfactants include, but are not limited to, TWEEN™ (e.g., TWEEN 80) and surfactants sold under the tradename PLURONIC™.

In certain formulations, a surfactant is not included, but is included in others. If added to the formulation, the concentration of the surfactant can vary, but generally ranges from about 0.01 - 0.5% (w/v) in liquid formulations. For certain formulations, the surfactant concentration ranges from about 0.01 - 0.1% (w/v) or from 0.02-0.1% (w/v). Various formulations thus contain at least 0.01, 0.025, 0.05, 0.075, 0.1, 0.2, 0.3, or 0.4% surfactant (w/v), but less than about 0.5% (w/v). The concentration should not exceed a level that results in adverse effects on lung tissue when administered.

### E. Antioxidant

"Antioxidant" as used herein has its general meaning in the art. It refers generally to an agent that inhibits oxidation through oxidative processes, specifically of amino acid residues in AAT. The purpose of the antioxidant in the formulation is to prevent oxidation of amino acids that are important for protein activity and/or stability, for example. AAT, for instance, includes an important methionine residue which, if oxidized, results in decreased activity. A number of antioxidants utilized in pharmaceutical compositions can be incorporated into the formulation. Exemplary antioxidants include methionine, glutathione, cysteine, and ascorbic acid, but other antioxidants known in the art can be used as well. Some formulations, for example, include N-acetyl cysteine.

In liquid form, the antioxidant level is generally about 1-10 mM. Various formulations thus contain about 1, 2, 4, 5, 6, 8 or 10 mM antioxidant, although certain formulations can contain somewhat more or less.

### F. Buffer

Various buffers that can be nebulized or converted into an aerosol from dry powder and that are compatible with lung tissue are typically included in the formulations. The buffer should also provide buffering capacity at a pH between about pH 6.5-7.5, as pH values in this range are compatible with the lungs. Certain formulations have a pH between 7-7.5; other compositions have a pH between 7.2 - 7.5. Other formulations have somewhat lower pH, such as 6.6-6.8. The formulations in some instances include phosphate buffer because it has good buffering capacity over the desired pH range and is relatively inexpensive. Other buffers (e.g., TRIS), however, can also be used.

### G. Additional Optional Components

Certain formulations can contain various optional components to address various other symptoms that are sometimes associated with AAT deficiency. Some compositions contain other serine protease inhibitors such as listed in the following section. Other compositions can include agents effective to ameliorate inflammation that is associated with the degradation of lung connective tissue. Such anti-inflammatory agents can be selected from any of those generally known in the art, provided they are compatible with lung tissue, can be nebulized or converted into an aerosol. For instance, certain formulations can include a corticosteroid. Specific examples of such compounds include, but are not limited to, hydrocortisone, dexamethasone, triamcinolone acetonide, prednisone, beclometasone valerate, hydrocortisone valerate, and halocinonide.

### H. Powder Compositions

Liquid formulations with the compositions described herein can be lyophilized to form a powder. Thus powder formulations prepared by lyophilizing liquid formulations such as described herein are also provided. As discussed in greater detail below, such compositions can be administered as a dry powder aerosol. Alternatively, the powder can be rehydrated to form a liquid formulation that can be administered according to the various options described below.

### I. Variations

Although the foregoing discussion has focused on AAT formulations, the components just described can be compounded in the concentrations listed above to prepare formulations with a variety of other proteins. The formulation can contain essentially any therapeutic protein that can be nebulized when dissolved in aqueous solution or converted into an aerosol from a dry powder. So, for instance, the protein can be a member from the general class of plasma serum protease inhibitors to which AAT belongs, including various serine protease inhibitors. Exemplary serine proteases include, but are not limited to, those that can be obtained from mast cells, neutrophils, eosinophils and/or basophils, such as elastase, cathepsin-G, kinins, tumor necrosis factor, collagenase, chymotrypsin, tryptase, kalikrein, and chymase. Specific examples of serine protease inhibitors that can be included in the formulations include, but are not limited to, C-reactive protein, alpha cysteine protease inhibitors, C-1-inhibitor, alpha 2-macroglobulin, alpha 2-antiplasmin, serine amyloid A protein, secretory leucocyte protease inhibitor, bronchial mucous inhibitor and inter-alpha-trypsin inhibitor.

### J. Shelf Life

The formulations that are provided can retain substantially their original activity for at least 2, 4, 6, 8, 10, 12 or more months. For example, lyophilized formulations stored at -70 °C or 25 °C can typically retain at least 70-95% of their activity when stored for 6 months. Some lyophilized compositions retain 90%, 95%, 98% or higher activity when stored for 6 months or longer. Even at higher temperatures such as 40-50 °C, lyophilized compositions retain at least 85-80% of their original activity over a six month period. Certain compositions were found to be 5000-8000 times as stable as unstablized compositions. Compositions can be stable and suitable for pharmaceutical use for at least 6 months, 12 months, 18 months, 24 months or even longer.

### IV. Formulation and Administration

The components of the formulation (e.g., protein, carbohydrate, surfactant, antioxidant and/or buffer) are mixed together according to standard methods In the art. In some Instances, for example, a concentrated protein solution is mixed with a volume of solution containing the other components of the formulation. As a specific example, 4 volumes of concentrated protein solution (e.g., 60-65 mg/ml) is mixed with 1 volume of a 5 x mixture of the other components to obtain a final solution that has a protein concentration of about 48-52 mg/ml and with the concentration of the other components In the ranges specified supra. This resulting solution is filtered through a 0.2 micron filter to sterilize the solution and subsequently transferred Into sterilized glass vials. These vials are sealed with sterile stoppers and then lyophilized. When ready for use, the composition can be reconstituted by adding sterilized water. Further details are provided in the Example below.

The components used to formulate the pharmaceutical compositions are preferably of high purity and are substantially free of potentially harmful contaminants (e.g., at least National Food (NF) grade, generally at least analytical grade, and more typically at least pharmaceutical grade). To the extent that a given compound must be synthesized prior to use, the resulting product Is typically substantially free of any potentially toxic agents, particularly any endotoxins, which may be present during the synthesis or purification process. Compositions are also made under GMP conditions.

If the formulation is to be delivered as a dry powder aerosol, the powdered formulation is typically sized to allow for transport of the powder to the deep lung. Guidance regarding appropriate particle size and methods for preparing dry powder AAT formulations is provided in U.S. Patent No. 5,993,783.

### V. Treatment Methods

The AAT formulations that are provided can be used In therapeutic and prophylactic treatment of a patient with an AAT deficiency or of a patient susceptible to such a deficiency. Such methods are useful in ameliorating or preventing the harmful effects associated with AAT deficiencies.

The term "treatment" as used herein refers to both therapeutic and/or prophylactic treatment. The term is also meant broadly to refer to reduction in severity and/or frequency of symptoms, elimination of symptoms and/or underlying cause, prevention of the occurrence of symptoms and/or their underlying cause, and/or improvement or remediation of damage. The term "patient" as used herein is meant to refer generally to a mammalian patient that is AAT deficient or is at risk for such a deficiency. Often the treatment methods are used to treat humans. As discussed in the Background section, AAT deficiencies are correlated with genetic, environmental and life-style (e.g., smoking) factors.

AAT is generally administered in treatment methods in an "effective amount." This refers to a sufficient but nontoxic amount of the agent to provide the desired effect (e.g., reversal of AAT deficiency) in therapeutic treatment methods, the formulation/composition Is administered in a "therapeutically effective amount," which is an amount sufficient to slow or reverse an AAT deficiency, to eliminate the deficiency, or to remedy symptoms associated with an AAT deficiency. Such therapeutic methods are In some Instances continued until AAT levels In the lower respiratory tract (e.g., the lower lung) are at levels typical of individuals without an AAT deficiency. When prophylactic treatment Is administered, the formulation is administered In a "prophylactically effective amount." This in general is an amount sufficient to retard or prevent the occurrence of an AAT deficiency in a patient susceptible to an AAT deficiency (e.g., because of genetic or other risk factors).

In general, the composition is administered to a patient so it reaches the lower respiratory tract (i.e., the deep lung) of the patient. For liquid formulations this is typically accomplished by nebulizing the reconstituted liquid formulation according to established means in the art. The nebulized formulation can then be inhaled by the patient and thus transported Into the desired regions of the deep lung. Any of a variety of nebulizers known in the art can be utilized to nebulize the formulations. Exemplary nebulizers that can be used with certain of the formulation disclosed herein include, but are not limited to, those discussed in U.S. Patent Nos. 5,150,071; 4,198,969; 4,253,468; 4,301.970; 4,453,542; 4,150,071; and 4,620,870. Methods regarding the delivery of AAT formulations using nebulizers are discussed in U.S. Patent Nos. 5,618,786 and 5, 780,440,

If the formulation is a powder and is to be delivered as a dry powder, the nebulizers and methods discussed in U.S. Patent No. 5,993,783, for example, can be used.

As those of skill in the art will appreciate, the amount of AAT administered will depend upon various factors. Such factors include, but are not limited to the age and weight of the patient, frequency of administration, and the severity of the AAT deficiency (e.g., whether the treatment is prophylactic or therapeutic). Toxicity and therapeutic efficacy can be determined according to standard pharmaceutical procedures in tissue culture and/or experimental animal models, including, for instance, determining the LD₅₀ (the dose lethal to 50% of the population) and the ED₅₀ (the dose therapeutically effective in 50% of the population). In certain treatment methods, the AAT dosage ranges from about 0.1 mg/kg body weight/day to about 10 mg/kg body weight/day. In other instances, the dosage amount ranges from about 1 mg/kg body weight/day to about 5 mg/kg body weight/day. When patients with less severe symptoms are treated or in certain prophylactic methods, the dosage levels may be somewhat lower, ranging from about 5 µg/kg body weight/day to about 100 µg/kg body weight/day.

The formulations can be tailored by an attending physician according to various schedules depending upon the severity of the patient's AAT deficiency and the concentration of AAT in the formulation. In less severe cases or for prophylactic treatment, for instance, the formulation may only be administered every few days to once a day. More severe cases can require that the formulation be administered several times (e.g., 2-4 times) a day.

The formulations that are provided can be administered to treat essentially any pulmonary related disease that is responsive to AAT, such as various AAT-deficiency diseases. So, for example, because AAT is an inhibitor of elastase, cathepsin G and/or proteinase 3, the formulations can be used to treat pulmonary disease associated with the activity of these enzymes. Because AAT also inhibits the activation of neutrophils, mast cells and T-cells, the compositions can also be used to treat diseases associated with the activation of these cell types. Specific examples diseases that can be treated include pulmonary emphysema, asthma, adult respiratory distress syndrome, neonatal respiratory distress syndrome, sepsis syndrome and cystic fibrosis.

The following example is provided to illustrate certain aspects of the disclosed formulations and methods but should not be construed to limit the claimed invention.

### EXAMPLE

### I. Introduction

Proteins when removed from their natural environment can become subjected to a variety of conditions that can negatively affect their stability. Studies with aqueous solutions of AAT showed that within 7 days AAT activity can drop as much as 48% when stored at 45 °C. Because of the value of AAT as a therapeutic, there is a substantial need for stable AAT formulations.

To support screening of lyophilized formulations for rAAT, nine formulations were compounded, lyophilized and placed on stability for up to six months. Four excipients were included in the formulations: 1) trehalose (protein stabilizer), 2) Tween-80 (surfactant), 3) methionine (antioxidant), and 4) sodium phosphate (buffering agent). The nine formulations had varying levels of 1) rAAT protein, 5% and 10% (w/v), 2) trehalose, 0% to 5% (w/v), and 3) Tween-80, 0.0 % to 0.5%. All formulations included 10mM sodium phosphate and 5mM methionine. The utility of the formulation in preserving rAAT was evaluated by conducting assays for functional activity, total protein and aggregation (i.e., % monomer). The composition of these nine formulations is summarized in Table 1.

### II. Methods

### A. Buffer Preparation

All buffers required for compounding were prepared under standard laboratory conditions. At ambient temperature, reagents were weighed and added to deionized (DI) water. The solutions were mixed, adjusted to the required pH and brought up to the required volume with DI water. The solutions were then filtered thru 0.22 µ filter.

Typically, a 10mM sodium phosphate buffer, adjusted to pH 7.4, was prepared. Then, required amounts of trehalose, Tween-80 and methionine were added to the phosphate buffer and the solutions were mixed, adjusted to the required pH and brought up to the required volume with DI water. The solutions were then filtered thru a 0.22 µ filter.

### B. Thawing and Processing of rAAT Bulk Drug Substance (BDS)

The BDS was stored at -70°C and was allowed to thaw at about 30 °C. The BDS was used "as is", without further processing, for the production of formulations 1-3 and 4-5. However, a different lot was used for the production of formulations 6-9, which required a buffer exchange against 10 mM Sodium Phosphate, pH 7.4 and concentration from - 50 mg/ml to ~ 100 mg/ml. Both of these steps were accomplished by using Tangential Flow Filtration.

### C. Formulation of rAAT BDS and Lyophilization

All formulations were carried out under standard laboratory conditions, not under laminar flow hood. The formulation buffers were prepared and mixed with rAAT BDS under these conditions. All product solutions were prepared to achieve final composition according to Table 1.

After partial stoppering, temperature probes were placed inside the vials, at least one for each formulation. A regular thermocouple, Omega thermocouple, was used and secured inside the vials through a punched hole in the rubber stopper. The temperature of the chamber was room temperature or +5 °C when the trays were loaded. Once the trays were loaded, lyophilization cycles commenced.

### D. Post-lyophilization Testing (0-month)

The appearance of the cake was noted after lyophilization. Each vial was tested for presence of vacuum using the "spark test". Vials from each formulation were reconstituted with 2ml Water for Injection (WFI) and tested for total protein (Practical Approaches to Protein Formulation Development, Byeong Chang and Susan Henderson, Rational Design of Stable Protein Formulations: Theory and Practice, Edited by John F. Carpenter and Mark C. Manning, Pharmaceutical Biotechnology Volume 13, Kluwer Academic/Plenum Publishers (2002), p. 13), functional activity, and monomer content.

Each vial was labeled with a cryogenic label as it was taken out of the tray. The vials were then sealed using the lab sealer. All product vials with vacuum were placed in -70°C, +5 °C, +25 °C, +40 °C, +50 °C and +60 °C incubators for stability study. A minimum of 6 vials were prepared.

### E. Stability Testing

1. Sample vial pull: After 0-month testing, Formulations 1- 9, were placed on stability at 6 different temperatures, -70°C, +5°C, +25°C, +40°C, +50°C and +60°C. At the time points indicated in Tables 2A and 2B, sample vials were pulled from the appropriate stability chambers. Two vials were pulled from each storage temperature, and labeled #1 and #2. Each vial was labeled with the appropriate time point identification, in Months. Vials were allowed to equilibrate to room temperature before proceeding to the next step.

2. Reconstitution: Each vial was reconstituted with 5.0 ml of Water for Injection, WFI, obtained from a newly opened bottle. Each vial was gently swirled to allow complete dissolution. All vials were placed in an ice-water bath from this point on when not in use.

3. Making of 1.0 mg/ml dilutions for stability testing: The 1.0 mg/ml dilutions was made as follows: 500 µl of 20 mg/ml solution from each vial was added to 9.5 ml of 0.2M Tris•Cl, pH 8.0, in a 15 ml centrifuge tube. The protein concentration in each tube was approximately 1 mg/ml. Exact concentration was verified by A₂₈₀. The A₂₈₀ result was used to calculate the total protein in each reconstituted vial. Two 1 mg/ml dilutions were made from each sample vial.

4. Total Protein (A₂₈₀): The 2 sets of 1-mg/ml dilutions were used for this assay. Briefly, 0.2M Tris Cl, pH 8.0 was used as a blank. Then 100 µl of Tris was added to several wells on a UV microplate. Next, 100 µl of each 1 mg/ml sample was added to each of several wells, pre-defined on the software. Once all the samples were loaded on the plate, the plate was read at 280 nm and 320 nm. The data output was obtained as ABS₂₈₀, ABS₃₂₀ and ABS₂₈₀₋₃₂₀. It is the ABS₂₈₀₋₃₂₀ that was used for Total Protein and Activity calculations.

5. rAAT Activity Assay: The 2 sets of 1-mg/ml dilutions were used for the activity assay. The assay is based on the ability of rAAT and pancreatic porcine elastase (PPE) to form an equimolar covalent complex. A reference curve is generated from the optical response of the substrate in the presence of various amounts of PPE. The curve is utilized to calculate the functional activity of rAAT by determining percent of PPE-rAAT complex formed from a given amount of rAAT and PPE. Typically, this value is expressed in moles of PPE over moles of rAAT. The activity of recombinant alpha 1-antitrypsin is determined by incubating with varying concentrations of pancreatic porcine Elastase at 30°C for 30 minutes. During this incubation the rAAT binds with a portion of the PPE, this dependent on the dilution of rAAT tested. After this incubation period a chromogenic substrate (Suc-A-A-A-pNA) is added to the reaction mixture. The chromophore is cleaved by the PPE not bound by the rAAT. The released p-nitroaniline generates a yellow color measured spectrophotometrically at 405nm. Percent inhibition is determined using a PPE reference at varying concentrations and comparing the absorbance values obtained for the test samples. AAT activity can be measured as described in U.S. Patent 4,931,373.

6. rAAT Aggregation by SEC-HPLC/UV Assay: The 2 sets of 1mg/ml dilutions were used for the aggregation assay using size exclusion HPLC analysis. Peaks representing different aggregation states are detected and recorded as protein elutes from the column. The major HPLC parameters were: a) protein Concentration: 1 mg/ml; b) Dilution Buffer: 0.2 M Tris Cl, pH 8.0; c) Mobile Phase: 200 mM KCI, 20 mM Na₂HPO₄, 5 mM NaCitrate, pH 7.5, d) HPLC Column: TSK-GEL G3000 SWXL, 7.8 mm x 300 mm, 5 µm particle size, Toso Haas part # 08541, with Guard SWxl guard column, 6.0 mm x 40 mm, 7 µm particle size, TosoHaas part # 08543; e) Pump Gradient: Isocratic; f) Injection Volume: 100 µl; g) Flow Rate: 0.3 ml/min; h) Detector: Variable Wavelength Detector, VWD; and f) Detection Wavelength: 280 nm.

### III. Stability Assessment

All formulations, F1 - F9, were placed on stability for 0 - 6 months. The formulations were placed at varying temperature levels as described supra. Total protein, activity and aggregation of each formulation was determined at the appropriate temperatures and storage conditions. Samples that are designated as n/a indicate that a stability pull and subsequent testing was not performed.

### A. Protein and Activity Recovery

The total protein and activity of each of the nine formulations, F1 - F9, was measured at temperatures of -70 °C, 5 °C, 25 °C, 40 °C. 50 °C and 60 °C for storage of 0 to 6 months. The ABS₂₈₀₋₃₂₀ value was tabulated. The total protein of the formulations was determined by dividing this value by the extinction coefficient of rAAT (0.485) and multiplying the quotient by 50 to account for a 50-fold dilution factor. The activity of each formulation is shown in Tables 2A and 2B.

### B. Monomer Content Recovery

The aggregation of each of the nine formulations, F1 - F9, was measured at temperatures of -70 °C, 5 °C, 25 °C, 40 °C. 50 °C and 60 °C for storage of 0 to 6 months. The HPLC chromatograms produced percentages for both the aggregation and monomer content of each formulation.

### C. Effect of rAAT Concentration

The effect of rAAT concentration on the stability of two formulations was examined by determining the monomer content of formulations F1 and F2. Both formulations contained only rAAT protein in 10 mM sodium phosphate, pH 7.4. Formulation F1 contained 50 mg/mL rAAT, while formulation F2 contained 100 mg/mL rAAT.

### D. Effect of Tween-80 Concentration

The effect of the concentration of tween-80 on the stability of rAAT formulations was examined by determining the monomer content of formulations F5, F6, F7 and F8. Each formulation contained 50 mg/mL rAAT, 2.5 % trehalose, 5 mM methionine and varying percentages of tween-80 in 10 mM sodium phosphate, pH 7.4.

### E. Effect of Trehalose Concentration

The effect of the concentration of trehalose on the stability of rAAT formulations was examined by determining the monomer content of formulations F1 - F9. Each formulation contained varying percentages of trehalose (see Table 1 for the individual compositions). The monomer contents of these formulations as a function of their percentage of trehalose can be found in Tables 3A and 3B.

### IV. Discussion

### A. Stability Assessment

### 1. Protein Recovery

All nine formulations showed a lack of apparent change in total protein in their 0 to 6 month storage at the temperatures indicated. Variation exists (within 15 %) between the nine formulations; however, this is likely due to the inherent variability of the assay and possibly associated with the use of a microwell plate.

### 2 Activity Recovery

The storage temperatures of -70 °C, 5 °C, 25 °C, 40 °C and 50 °C showed activity levels that were consistent over the 6-month storage period. For example, formulations F5 and F6, stored at 25 °C, showed 81.7% and 78.1% for 0-month storage and 77.2% and 82.4% for 6-month storage activity levels, respectively. There was no apparent change in the activities of any of the formulations stored for 0 - 6 months at -70 °C to 50 °C. Any variation that existed was possibly associated with an inherent variability of the assay and possibly associated with the use of a microwell plate.

The only apparent change in activity was associated with formulations F2, F4 and F9, stored at 60 °C. Formulation F2 showed a 23 % decrease in activity from 62.4 % inhibition (0 month) to 39.4 % at 3 months of storage. Similar results were shown with formulation F4 in which the 0-month sample had an activity of 64.6 %, while the sample, stored for 4 months at 60 °C had 39.7 %. The common link between these two formulations is that neither contained trehalose. There may be a possible link between the lack of trehalose present in the formulation and the apparent decrease in activity from 0 to 4 months of storage at 60 °C. Formulation F9 contained 1 % trehalose while the remaining six formulations contained 2.5 %. The activity of F9 had a possible decrease from 76.7 % inhibition at 0 months of 60 °C storage to 49.1 % at 6 months of storage. In comparison to formulations F6, F7 and F8 that had activities of 78.1 %, 81.6 % and 78.9 % at 0 months and 69.7 %, 72.6 % and 66.1 % at 6 months of storage, respectively, formulation F9 showed a greater decrease at 6 months of storage. This indicates a possible association between the decreased activity and the lower percent of trehalose present in the formulation

### B. Monomer Content Recovery

### 1. Effect of rAAT Concentration

Formulation F1 contained 50 mg/mL rAAT while Formulation F2 contained 100 mg/mL rAAT, both in 10 mM sodium phosphate, pH 7.4. There was no apparent difference in the monomer content of formulations F1 and F2 at a storage time of 0 to 3 months. For example, formulation F1 contained a percent monomer of 49.8 upon storage for 3 months at 60 °C. Under the same conditions, formulation F2 contained 44.0 % monomer, with both formulations starting with a 95.5 % monomer content at 0 months storage. Therefore, the higher concentration of protein in formulation F2 as compared to F1 did not have a significant effect on the stability of the formulation.

### 2. Effect of Tween-80 Concentration

The effect of Tween-80 concentration on the stability of four rAAT formulations was investigated by determining the percent monomer content of each formulation with varying concentrations of Tween-80. Tween-80 had no apparent effect on the stability of the formulations, F5, F6, F7 and F8. The percent monomer was consistent between all formulations under all storage conditions. For example, formulation F6 with 0 % tween-80 showed an 84.2 % monomer upon storage at 60 °C for 6 months, while formulation F7 (0.02 % tween-80) and F8 (0.10 % tween-80) showed monomer contents of 82.0 % and 81.4 %, respectively.

### 3. Effect of Trehalose Concentration

To investigate the effect of trehalose concentration on the stability of the rAAT formulations, the monomer content of all nine formulations, F1 - F9, were analyzed. Formulations F3 - F9 showed no apparent change in monomer content from their initial values in storage for six months and formulations F1 and F2 at 3 months storage at -70 °C, 5 °C and 25 °C (Table 3A). The first observable change occurs at a 40 °C storage temperature, with the most dramatic effects at 60°C. The monomer content of the formulations F1, F2 and F4 that contained 0 % trehalose decreased from 95.5 % and 94.1 % at 0 month storage to 59.4%, 53.6 %, and 60.5 % after 2 months of storage at 60 °C, respectively (Table 3B). This is a definite decrease in the monomer content as compared to the remaining formulations.

Formulation F9, containing 1.0 % trehalose, also showed a small decrease in the percent monomer in which the original percentage, 98.0 %, decreased to 57.8 % after storage for 6 months time. This was a slightly higher decrease in monomer content than those formulations that contained 2.5 % or 5.0 % trehalose. Those formulations, F6, F7 and F8, decreased to only 84.2 %, 82.0 % and 81.4 % monomer after six months of storage from their initial values of 98.3 %, 98.1 %, and 97.4 %, respectively. Therefore, the formulations that contained a minimum of 2.5 % trehalose demonstrated the most stability in maintaining a lesser decrease in monomer content upon storage for 6 months at 60 °C.

### V. Conclusion

The formulations tested all showed significant improvements in stability relative to AAT compositions without stabilizers. Inclusion of a carbohydrate such as trehalose had the most appreciable beneficial effect on stability of the various factors tested. Inclusion of a surfactant had a lesser effect on stability, as did increased AAT protein concentrations. Formulations containing 2.5% trehalose behave similar to the one formulation containing 5%. Since minimal amount of excipient is ideal In product development, a formulation having a composition of about 2.5% carbohydrate (e.g., trehalose) along with 5% rAAT, 0.1% or 0.02% surfactant (e.g., TWEEN-80), 5mM antioxidant (e.g., mothionine) and 10mM buffer (e.g., sodium phosphate (pH 7.4)), provides a good base formulation.

It is understood that the examples and embodiments described herein are for illustrative purposes only and that various modifications or changes In light thereof will be suggested to persons skliled In the art and are to be included within the scope of the appended claims.

**Table 1. The Composition of rAAT Formulations 1-9 (F1-F9).**

| **Formulation** | **rAAT (mg/ml)** | **Trehalose (%)** | **Methionine (mM)** | **Tween-80 (%)** |
|---|---|---|---|---|
| F1 | 50 | 0 | 0 | 0.0 |
| F2 | 100 | 0 | 0 | 0.0 |
| F3 | 50 | 5 | 5 | 0.5 |
| F4 | 50 | 0 | 5 | 0.5 |
| F5 | 50 | 2.5 | 5 | 0.5 |
| F6 | 50 | 2.5 | 5 | 0.0 |
| F7 | 50 | 2.5 | 5 | 0.02 |
| F8 | 50 | 2.5 | 5 | 0.1 |
| F9 | 50 | 1 | 5 | 0.1 |

| | | | | |
|---|---|---|---|---|
| Note: All formulations were made in 10 mM Sodium Phosphate, pH 7.4. | | | | |

**Table 2A. Activity (%) of Formulations F1-F9 at -70°C, 5°C and 25 °C for 0 to 6 Month Storage**

| **-70°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | 70.9 | 89.9 | 69.3 | 77.0 | N/A | 81.2 | N/A |
| F2 | 62.4 | 71.4 | 69.4 | 84.4 | 67.2 | N/A | N/A | N/A |
| F3 | 54.6 | 75.0 | 76.6 | 85.7 | 76.5 | N/A | 82.6 | 89.0 |
| F4 | 64.6 | 82.3 | 67.7 | 74.3 | N/A | 90.2 | 76.5 | 73.2 |
| F5 | 81.7 | 84.0 | 70.3 | 76.0 | N/A | 88.9 | 81.3 | 82.0 |
| F6 | 77.1 | N/A | 80.4 | 74.6 | 90.9 | N/A | N/A | 87.7 |
| F7 | 81.6 | N/A | 102.1 | 79.4 | 79.3 | N/A | N/A | 76.9 |
| F8 | 78.9 | N/A | 78.2 | 86.4 | 81.4 | N/A | N/A | 85.2 |
| F9 | 76.7 | N/A | 72.4 | 93.3 | 83.0 | N/A | N/A | 88.0 |

| **5°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | N/A | N/A | N/A | 76.9 | N/A | 80.3 | N/A |
| F2 | 62.4 | N/A | N/A | N/A | 69.8 | N/A | N/A | N/A |
| F3 | 54.6 | N/A | N/A | N/A | 68.2 | N/A | 80.2 | 90.8 |
| F4 | 64.6 | N/A | N/A | N/A | N/A | 69.6 | 68.5 | 73.3 |
| F5 | 81.7 | N/A | N/A | N/A | N/A | 86.0 | 119.9 | 81.3 |
| F6 | 78.1 | N/A | N/A | 85.9 | 78.3 | N/A | N/A | 85.6 |
| F7 | 81.6 | N/A | N/A | 84.4 | 81.7 | N/A | N/A | 86.3 |
| F8 | 78.9 | N/A | N/A | 85.9 | 83.4 | N/A | N/A | 81.7 |
| F9 | 76.7 | N/A | N/A | 74.7 | 76.9 | N/A | N/A | 73.9 |

| **25°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | N/A | N/A | N/A | 75.1 | N/A | 73.9 | N/A |
| F2 | 62.4 | N/A | N/A | N/A | 62.3 | N/A | N/A | N/A |
| F3 | 54.6 | N/A | N/A | N/A | 71.9 | N/A | 76.4 | 96.6 |
| F4 | 64.6 | N/A | N/A | N/A | N/A | 78.1 | 64.3 | 57.6 |
| F5 | 81.7 | N/A | N/A | N/A | N/A | 80.0 | 76.5 | 77.2 |
| F6 | 78.1 | N/A | N/A | 72.1 | 87.0 | N/A | N/A | 82.4 |
| F7 | 81.6 | N/A | N/A | 87.3 | 75.3 | N/A | N/A | 77.1 |
| F8 | 78.9 | N/A | N/A | 80.0 | 88.7 | N/A | N/A | 76.9 |
| F9 | 76.7 | N/A | N/A | 85.4 | 81.0 | N/A | N/A | 88.0 |

**Table 2B. Activity (%) of Formulations F1-F9 at 40°C, 50°C and 60°C for 0 to 6 Month Storage**

| **40°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | N/A | 62.6 | 66.0 | 79.7 | N/A | 69.9 | N/A |
| F2 | 62.4 | N/A | 65.3 | 82.4 | 57.7 | N/A | N/A | N/A |
| F3 | 54.6 | N/A | 74.7 | 91.8 | 57.1 | N/A | 73.4 | 67.4 |
| F4 | 64.6 | N/A | 59.8 | 62.6 | N/A | 67.6 | 59.8 | 63.2 |
| F5 | 81.7 | N/A | 69.1 | 82.4 | N/A | 92.8 | 669.2 | 72.1 |
| F6 | 78.1 | N/A | 79.7 | 77.0 | 71.8 | N/A | N/A | 81.7 |
| F7 | 81.6 | N/A | 80.5 | 87.5 | 85.6 | N/A | N/A | 80.4 |
| F8 | 78.9 | N/A | 70.1 | 81.9 | 79.8 | N/A | N/A | 75.8 |
| F9 | 76.7 | N/A | 68.1 | 60.8 | 72.8 | N/A | N/A | 71.3 |

| **50°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | 63.5 | 62.3 | 61.5 | 63.1 | N/A | N/A | N/A |
| F2 | 62.4 | 65.5 | 63.7 | 76.0 | 73.0 | N/A | N/A | N/A |
| F3 | 54.6 | 67.1 | 70.4 | 85.8 | 71.7 | N/A | N/A | N/A |
| F4 | 64.6 | 70.3 | 56.0 | 62.6 | N/A | 70.5 | N/A | N/A |
| F5 | 81.7 | 80.2 | 67.9 | 81.6 | N/A | 78.8 | N/A | N/A |
| F6 | 78.1 | N/A | 77.7 | 71.5 | 79.7 | N/A | N/A | 81.4 |
| F7 | 81.6 | N/A | 82.6 | 78.8 | 78.5 | N/A | N/A | 74.4 |
| F8 | 78.9 | N/A | 72.2 | 81.1 | 85.0 | N/A | N/A | N/A |
| F9 | 76.7 | N/A | 72.4 | 85.6 | 69.8 | N/A | N/A | 71.3 |

| **60°C** | | | | | | | | |
|---|---|---|---|---|---|---|---|---|
| **Formulation** | **Time, Months** | | | | | | | |
| | **0** | **0.5** | **1** | **2** | **3** | **4** | **5** | **6** |
| F1 | 59.0 | 60.6 | 51.5 | 72.2 | 51.4 | N/A | N/A | N/A |
| F2 | 62.4 | 69.2 | 58.6 | 58.9 | 39.4 | N/A | N/A | N/A |
| F3 | 54.6 | 70.9 | 64.5 | 85.1 | 64.5 | N/A | N/A | N/A |
| F4 | 64.6 | 71.2 | 54.6 | 53.1 | N/A | 39.7 | N/A | N/A |
| F5 | 81.7 | 78.1 | 63.9 | 94.7 | N/A | 73.7 | N/A | N/A |
| F6 | 78.1 | N/A | 76.5 | 78.9 | 72.8 | N/A | N/A | 69.7 |
| F7 | 81.6 | N/A | 79.3 | 77.9 | 71.8 | N/A | N/A | 72.6 |
| F8 | 78.9 | N/A | 70.0 | 85.4 | 77.1 | N/A | N/A | 66.1 |
| F9 | 76.7 | N/A | 64.6 | 58.4 | 65.8 | N/A | N/A | 49.1 |

**Table 3A. Effect of Trehalose Concentration on Monomer Content (%) at Temperatures of -70 °C, 5°C and 25°C for 0 to 6 Month Storage**

| | | | **Months** | | | | |
|---|---|---|---|---|---|---|---|
| **Formulation Number^{A}** | **Temperature** | **Trehalose** | **0** | **1** | **2** | **3** | **6** |
| 1 | -70°C | 0.0% | 95.5 | 95.8 | 95.6 | 94.4 | ND |
| 2 | | 0.0% | ND | 95.2 | 94.5 | 95.1 | ND |
| 4 | | 0.0% | 94.1 | 96.7 | 96.0 | NA | 93.7 |
| 9 | | 1.0% | 98.0 | 97.5 | 97.4 | 96.8 | 96.6 |
| 6 | | 2.5% | 98.3 | 97.9 | 97.9 | 97.5 | 97.1 |
| 7 | | 2.5% | 98.1 | 97.7 | 97.8 | 97.2 | 97.2 |
| 8 | | 2.5% | 97.4 | 97.6 | 97.8 | 96.4 | 93.6 |
| 5 | | 2.5% | 96.2 | 96.9 | 96.6 | NA | 96.6 |
| 3 | | 5.0% | 93.4 | 95.4 | 93.9 | 95.0 | 93.7 |
| 1 | 5°C | 0.0% | 95.5 | ND | ND | 95.1 | ND |
| 2 | | 0.0% | ND | ND | ND | 94.5 | ND |
| 4 | | 0.0% | 94.1 | ND | ND | NA | 95.1 |
| 9 | | 1.0% | 98.0 | ND | 97.5 | 96.3 | 96.0 |
| 6 | | 2.5% | 98.3 | ND | 97.9 | 97.2 | 96.9 |
| 7 | | 2.5% | 98.1 | ND | 97.8 | 97.2 | 97.1 |
| 8 | | 2.5% | 97.4 | ND | 97.8 | 96.6 | 96.6 |
| 5 | | 2.5% | 96.2 | ND | ND | ND | 95.7 |
| 3 | | 5.0% | 93.4 | ND | ND | 92.9 | 95.6 |
| 1 | 25°C | 0.0% | 95.5 | ND | ND | 89.5 | ND |
| 2 | | 0.0% | ND | ND | ND | 92.0 | ND |
| 4 | | 0.0% | 94.1 | ND | ND | ND | 87.5 |
| 9 | | 1.0% | 98.0 | ND | 96.2 | 94.9 | 93.0 |
| 6 | | 2.5% | 98.3 | ND | 97.5 | 96.6 | 96.1 |
| 7 | | 2.5% | 98.1 | ND | 97.7 | 96.3 | 95.6 |
| 8 | | 2.5% | 97.4 | ND | 97.1 | 96.0 | 95.5 |
| 5 | | 2.5% | 96.2 | ND | ND | ND | 95.1 |
| 3 | | 5.0% | 93.4 | ND | ND | 92.8 | 94.2 |

| | | | | | | | |
|---|---|---|---|---|---|---|---|
| ^{A}See Table 1 for the formulation compositions. | | | | | | | |

**Table 3B Effect of Trehalose Concentration on Monomer Content (%) at Temperatures of 40 °C, 50 °C and 60 °C for 0 to 6 Month Storage**

| | | | **Months** | | | | |
|---|---|---|---|---|---|---|---|
| **Formulation Number^{A}** | **Temperature** | **Trehalose** | **0** | **1** | **2** | **3** | **6** |
| 1 | 40°C | 0.0% | 95.5 | 91.4 | 89.1 | 81.2 | ND |
| 2 | | 0.0% | ND | 90.5 | 86.2 | 80.8 | ND |
| 4 | | 0.0% | 94.1 | 92.4 | 86.7 | NA | 80.2 |
| 9 | | 1.0% | 98.0 | 94.5 | 90.0 | 89.5 | 86.1 |
| 6 | | 2.5% | 98.3 | 96.6 | 96.2 | 95.1 | 92.7 |
| 7 | | 2.5% | 98.1 | 96.6 | 96.1 | 94.6 | 93.6 |
| 8 | | 2.5% | 97.4 | 96.4 | 95.9 | 93.9 | 92.8 |
| 5 | | 2.5% | 96.2 | 95.7 | 95.8 | NA | 90.1 |
| 3 | | 5.0% | 93.4 | 95.4 | 94.4 | 93.6 | 92.8 |
| 1 | 50°C | 0.0% | 95.5 | 85.4 | 78.8 | 69.0 | ND |
| 2 | | 0.0% | ND | 81.9 | 71.6 | 64.3 | ND |
| 4 | | 0.0% | 94.1 | 85.1 | 79.5 | NA | NA |
| 9 | | 1.0% | 98.0 | 91.5 | 90.0 | 83.5 | 75.9 |
| 6 | | 2.5% | 98.3 | 95.7 | 95.1 | 92.2 | 89.6 |
| 7 | | 2.5% | 98.1 | 95.6 | 94.5 | 92.1 | 89.4 |
| 8 | | 2.5% | 97.4 | 94.6 | 94.8 | 90.6 | NA |
| 5 | | 2.5% | 96.2 | 94.0 | 93.2 | NA | NA |
| 3 | | 5.0% | 93.4 | 94.2 | 93.3 | 90.3 | ND |
| 1 | 60°C | 0.0% | 95.5 | 80.2 | 59.4 | 49.8 | ND |
| 2 | | 0.0% | ND | 74.4 | 53.6 | 44.3 | ND |
| 4 | | 0.0% | 94.1 | 73.9 | 60.5 | NA | NA |
| 9 | | 1.0% | 98.0 | 85.6 | 81.5 | 71.7 | 57.8 |
| 6 | | 2.5% | 98.3 | 94.6 | 93.3 | 88.4 | 84.2 |
| 7 | | 2.5% | 98.1 | 94.1 | 92.9 | 88.5 | 82.0 |
| 8 | | 2.5% | 97.4 | 93.0 | 89.2 | 87.3 | 81.4 |
| 5 | | 2.5% | 96.2 | 89.9 | 90.9 | NA | NA |
| 3 | | 5.0% | 93.4 | 91.1 | 90.2 | 86.3 | ND |

| | | | | | | | |
|---|---|---|---|---|---|---|---|
| ^{A}See Table 1 for the formulation compositions. | | | | | | | |

### REFERENCES

Practical Approaches to Protein Formulation Development, Byeong Chang and Susan Henderson, Rational Design of Stable Protein Formulations: Theory and Practice, Edited by John F. Carpenter and Mark C. Manning, Pharmaceutical Biotechnology Volume 13, Kluwer Academic/Plenum Publishers (2002), p. 13
Development and Manufacture of Protein Pharmaceuticals, Edited by Steven Nail and Michael J. Akers, Pharmaceutical Biotechnology Volume 14, Kluwer Academic/Plenum Publishers (2002), pp. 65 - 66.
Lyophilization: Introduction and Basic Principles, Thomas A. Jennings, Interpharm Press (1999), pp. 43, 261 - 279.
Stabilization of Protein Pharmaceuticals in Freeze-dried Formulations, Ken-ichi Izutsu and Sumie Yoshioka, Stabilization of Freeze-dried Protein Pharmaceuticals*.*
Surfactant-Protein Interactions, Theodore W. Randolph, LaToya S. Jones, Rational Design of Stable Protein Formulations: Theory and Practice, Edited by John F. Carpenter and Mark C. Manning, Pharmaceutical Biotechnology Volume 13, Kluwer Academic/Plenum Publishers (2002), p. 13
Handbook of Pharmaceutical Additives 2nd Edition, Compiled by Michael and Irene Ash, Synapse Information Resources Inc. (2002), pp. 797 - 798

### SEQUENCE LISTING

SEQ ID NO:1 Amino acid sequence for alpha 1-antitrypsin

1 mpssvswgil llaglcclvp vslaedpqgd aaqktdtshh dqdhptfnki tpnlaefafs
61 lyrqlahqsn stniffspvs iatafamlsl gtkadthdei leglnfnlte ipeaqihegf
121 qellrtlnqp dsqlqlttgn glflseglkl vdkfledvkk lyhseaftvn fgdteeakkq
181 indyvekgtq gkivdlvkel drdtvfalvn yiffkgkwer pfevkdteee dfhvdqvttv
241 kvpmmkrlgm fniqhckkls swvllmkylg nataifflpd egklqhlene lthdiitkfl
301 enedrrsasl hlpklsitgt ydlksvlgql gitkvfsnga dlsgvteeap lklskavhka
361 vltidekgte aagamfleai pmsirpevkf nkpfvflmie qntksplfmg kvvnptqk

## Claims

1. A pharmaceutical composition formulated as a powder, comprising an alpha 1-antitrypsin (AAT), trehalose, a surfactant and an antioxidant, wherein the AAT is:
(a) a native AAT;
(b) a recombinant AAT; or
(c) an AAT variant that has at least 85% amino acid sequence identity to SEQ ID NO: 1 and can inhibit elastase.

2. The composition of claim 1, wherein the composition is in a form suitable for administration to a patient via inhalation therapy.

3. The composition of any one of the preceding claims, wherein the antioxidant is selected from methionine, glutathione, cysteine, ascorbic acid and N-acetyl cysteine.

4. The composition of claim 1, wherein the AAT, trehalose, surfactant and antioxidant are present in amounts such that if the powder is solubilized in aqueous solution for administration to a patient the AAT concentration is 1-100 mg/ml, the trehalose concentration is 1-5% (w/v), the surfactant concentration is 0.01-0.5% (w/v), and the antioxidant concentration is 1-10 mM.

5. The composition of claim 4, wherein the AAT concentration is 10-50 mg/ml.

6. The composition of claim 1, further comprising a buffer and wherein
(a) the antioxidant is methionine; and
(b) the AAT, trehalose, surfactant and methionine are present in amounts such that if the powder is solubilized in aqueous solution for administration to a patient (i) the AAT concentration is 10-50 mg/ml, (ii) the trehalose concentration is 10-50 mg/ml, (iii) the surfactant concentration is 0.01-0.5% (w/v), and (iv) the methionine concentration is 1-10 mM.

7. The composition of claim 1, comprising a recombinant alpha 1-antitrypsin (AAT), wherein the AAT/carbohydrate ratio (weight:weight) is 1:1 to 5:1.

8. The composition of claim 7, wherein the ratio is 1:1 to 2:1.

9. The composition of claim 7, wherein the surfactant is Polysorbate 80 and the antioxidant is methionine.

10. The composition of any one of claims 7 to 9, wherein the composition is formulated as a solid.

11. The composition of any one of the preceding claims, wherein the AAT is a native AAT.

12. The composition of any one of claims 1 to 10, wherein the AAT is a recombinant AAT.

13. The composition of any one of claims 1 to 10, wherein the AAT is an AAT variant that has at least 85% amino acid sequence identity to SEQ ID NO: 1 and can inhibit elastase.

14. The composition of any one of claims 1 to 10, wherein the AAT is glycosylated.

15. The composition of any one of claims 1 to 10, wherein the AAT is unglycosylated

16. The composition according to any one of claims 1 to 15 for use in a method for the treatment of a pulmonary disease associated with alpha 1-antitrypsin (AAT) deficiency, wherein the composition is to be administered to the lungs of a patient.

17. The composition for use in the treatment of a pulmonary disease associated of any one with alpha 1-antitespsin (AAT) deficiency of claim 16, wherein the composition is to be administered by inhalation.

18. The composition for use in the treatment of a pulmonary disease associated of any one with alpha 1-antitespsin (AAT) deficiency of claim 17, wherein the composition is a solid and is to be administered by converting the solid into a aerosol for inhalation by the patient.

19. The composition for use in the treatment of a pulmonary disease associated of any one with alpha 1-antitespsin (AAT) deficiency of claims 16 to 18, wherein the disease is a pulmonary disease associated with the activity of elastase, cathepsin G and/or proteinase 3.

20. The composition for use in the treatment of a pulmonary disease associated of any one with alpha 1-antitespsin (AAT) deficiency of claim 19, wherein the disease is a emphysema.

21. The composition for use in the treatment of a pulmonary disease associated of any one with alpha 1-antitespsin (AAT) deficiency of claims 16 to 18, wherein the disease is a pulmonary inflammatory disease associated with activation of neutrophils, mast cells or T-cells.

22. The composition for use in the treatment of a pulmonary disease associated of any one with alpha 1-antitespsin (AAT) deficiency of claim 21, wherein the disease is asthma.

23. The composition for use in the treatment of a pulmonary disease associated of any one with alpha 1-antitespsin (AAT) deficiency of claim 21, wherein the disease is adult respiratory distress syndrome, neonatal respiratory distress syndrome or sepsis syndrome.

24. The composition for use in the treatment of a pulmonary disease associated of any one with alpha 1-antitespsin (AAT) deficiency of any one of claims 16 to 18, wherein the patient is susceptible to the disease and the pharmaceutical composition is to be administered in a prophylactically effective amount.

25. The composition for use in the treatment of a pulmonary disease associated of any one with alpha 1-antitespsin (AAT) deficiency of any one of claims 16 to 18, wherein the patient has the disease and the pharmaceutical composition is to be administered in a therapeutically effective amount.

## Patentansprüche

1. Pharmazeutische, als Pulver formulierte Zusammensetzung, umfassend ein alpha-1-Antitrypsin (AAT), Trehalose, ein oberflächenaktives Mittel und ein Antioxidationsmittel, wobei das AAT ist:
(a) ein natives AAT;
(b) ein rekombinantes AAT; oder
(c) eine AAT-Variante, die mindestens 85 % Aminosäuresequenzidentität mit SEQ ID NO: 1 aufweist und Elastase hemmen kann.

2. Zusammensetzung nach Anspruch 1, wobei die Zusammensetzung in einer Form vorliegt, die zur Verabreichung an einen Patienten über eine Inhalationstherapie geeignet ist.

3. Zusammensetzung nach einem der vorhergehenden Ansprüche, wobei das Antioxidationsmittel aus Methionin, Glutathion, Cystein, Ascorbinsäure und N-Acetylcystein ausgewählt ist.

4. Zusammensetzung nach Anspruch 1, wobei das AAT, die Trehalose, das oberflächenaktive Mittel und das Antioxidationsmittel in solchen Mengen vorliegen, dass beim Lösen des Pulvers in einer wässrigen Lösung zur Verabreichung an einen Patienten die AAT-Konzentration 1-100 mg/ml beträgt, die Trehalosekonzentration 1-5 % (w/v) beträgt, die Konzentration des oberflächenaktiven Mittels 0,01 - 0,5 % (w/v) ist und die Konzentration des Antioxidationsmittels 1-10 mM ist.

5. Zusammensetzung nach Anspruch 4, wobei die AAT-Konzentration 10-50 mg/ml beträgt.

6. Zusammensetzung nach Anspruch 1, weiter umfassend einen Puffer und wobei
(a) das Antioxidationsmittel Methionin ist; und
(b) das AAT, die Trehalose, das oberflächenaktive Mittel und das Methionin in solchen Mengen vorliegen, dass beim Lösen des Pulvers in einer wässrigen Lösung zur Verabreichung an einen Patienten (i) die AAT-Konzentration 10-50 mg/ml beträgt, (ii) die Trehalose-Konzentration 10-50 mg/ml ist, (iii) die Konzentration des oberflächenaktiven Mittels 0,01-0,5 % (w/v) ist und (iv) die Methionin-Konzentration 1-10 mM beträgt.

7. Zusammensetzung nach Anspruch 1, umfassend ein rekombinantes alpha-1-Antitrypsin (AAT), wobei das AAT/Kohlenhydrat-Verhältnis (Gewicht:Gewicht) 1:1 bis 5:1 beträgt.

8. Zusammensetzung nach Anspruch 7, wobei das Verhältnis 1:1 bis 2:1 ist.

9. Zusammensetzung nach Anspruch 7, wobei das oberflächenaktive Mittel Polysorbat 80 und das Antioxidationsmittel Methionin ist.

10. Zusammensetzung nach einem der Ansprüche 7 bis 9, wobei die Zusammensetzung als Feststoff formuliert ist.

11. Zusammensetzung nach einem der vorhergehenden Ansprüche, wobei das AAT ein natives AAT ist.

12. Zusammensetzung nach einem der Ansprüche 1 bis 10, wobei das AAT ein rekombinantes AAT ist.

13. Zusammensetzung nach einem der Ansprüche 1 bis 10, wobei das AAT eine AAT-Variante ist, die mindestens 85 % Aminosäuresequenzidentität mit SEQ ID NO: 1 hat und Elastase hemmen kann.

14. Zusammensetzung nach einem der Ansprüche 1 bis 10, wobei das AAT glycosyliert ist.

15. Zusammensetzung nach einem der Ansprüche 1 bis 10, wobei das AAT unglycosyliert ist.

16. Zusammensetzung nach einem der Ansprüche 1 bis 15 zur Verwendung bei einem Verfahren zur Behandlung einer mit einem Mangel an alpha-1-Antitrypsin (AAT) im Zusammenhang stehenden Lungenkrankheit, wobei die Zusammensetzung an die Lungen eines Patienten zu verabreichen ist.

17. Zusammensetzung zur Verwendung bei der Behandlung einer mit einem Mangel an alpha-1-Antitrypsin (AAT) im Zusammenhang stehenden Lungenkrankheit nach Anspruch 16, wobei die Zusammensetzung durch Inhalation zu verabreichen ist.

18. Zusammensetzung zur Verwendung bei der Behandlung einer mit einem Mangel an alpha-1-Antitrypsin (AAT) im Zusammenhang stehenden Lungenkrankheit nach Anspruch 17, wobei die Zusammensetzung ein Feststoff ist und durch Umwandlung des Feststoffs in ein Aerosol zur Inhalation durch den Patienten zu verabreichen ist.

19. Zusammensetzung zur Verwendung bei der Behandlung einer mit einem Mangel an alpha-1-Antitrypsin (AAT) im Zusammenhang stehenden Lungenkrankheit nach einem der Ansprüche 16 bis 18, wobei die Krankheit eine Lungenkrankheit ist, die im Zusammenhang mit der Wirkung von Elastase, Kathepsin G und/oder Proteinase 3 steht.

20. Zusammensetzung zur Verwendung bei der Behandlung einer mit einem Mangel an alpha-1-Antitrypsin (AAT) im Zusammenhang stehenden Lungenkrankheit nach Anspruch 19, wobei die Krankheit ein Emphysem ist.

21. Zusammensetzung zur Verwendung bei der Behandlung einer mit einem Mangel an alpha-1-Antitrypsin (AAT) im Zusammenhang stehenden Lungenkrankheit nach einem der Ansprüche 16 bis 18, wobei die Krankheit eine entzündliche Lungenkrankheit ist, die im Zusammenhang mit der Aktivierung von Neutrophilen, Mastzellen oder T-Zellen steht.

22. Zusammensetzung zur Verwendung bei der Behandlung einer mit einem Mangel an alpha-1-Antitrypsin (AAT) im Zusammenhang stehenden Lungenkrankheit nach Anspruch 21, wobei die Krankheit Asthma ist.

23. Zusammensetzung zur Verwendung bei der Behandlung einer mit einem Mangel an alpha-1-Antitrypsin (AAT) im Zusammenhang stehenden Lungenkrankheit nach Anspruch 21, wobei die Krankheit ein Atemnotsyndrom bei Erwachsenen, ein neonatales Atemnotsyndrom oder ein Sepsissyndrom ist.

24. Zusammensetzung zur Verwendung bei der Behandlung einer mit einem Mangel an alpha-1-Antitrypsin (AAT) im Zusammenhang stehenden Lungenkrankheit nach einem der Ansprüche 16 bis 18, wobei der Patient für die Krankheit empfänglich ist und die pharmazeutische Zusammensetzung in einer prophylaktisch wirksamen Menge zu verabreichen ist.

25. Zusammensetzung zur Verwendung bei der Behandlung einer mit einem Mangel an alpha-1-Antitrypsin (AAT) im Zusammenhang stehenden Lungenkrankheit nach einem der Ansprüche 16 bis 18, wobei der Patient die Krankheit hat und die pharmazeutische Zusammensetzung in einer therapeutisch wirksamen Menge zu verabreichen ist.

## Revendications

1. Composition pharmaceutique formulée en une poudre, comprenant une alpha-1-antitrypsine (AAT), du tréhalose, un tensioactif et un anti-oxydant, dans laquelle l'alpha-1-antitrypsine est
a) une alpha-1-antitrypsine naturelle,
b) une alpha-1-antitrypsine recombinante,
c) ou un variant d'alpha-1-antitrypsine dont la séquence d'acides aminés est identique, pour au moins 85 %, à la Séquence N° 1 et qui peut inhiber l'élastase.

2. Composition conforme à la revendication 1, laquelle composition est sous une forme appropriée pour être administrée à un patient par inhalothérapie.

3. Composition conforme à l'une des revendications précédentes, pour laquelle l'anti-oxydant est choisi parmi la méthionine, le glutathion, la cystéine, l'acide ascorbique et la N-acétyl-cystéine.

4. Composition conforme à la revendication 1, dans laquelle l'alpha-1-antitrypsine, le tréhalose, le tensioactif et l'anti-oxydant se trouvent présents en des quantités telles que, quand on met la poudre en solution aqueuse pour l'administrer à un patient, la concentration d'alpha-1-antitrypsine vaut de 1 à 100 mg/mL, la concentration de tréhalose vaut de 1 à 5 % p/v, la concentration de tensioactif vaut de 0,01 à 0,5 % p/v, et la concentration d'anti-oxydant vaut de 1 à 10 mM.

5. Composition conforme à la revendication 4, avec laquelle la concentration d'alpha-1-antitrypsine vaut de 10 à 50 mg/mL.

6. Composition conforme à la revendication 1, qui comprend en outre un agent tampon et dans laquelle:
a) l'anti-oxydant est de la méthionine,
b) et l'alpha-1-antitrypsine, le tréhalose, le tensioactif et la méthionine se trouvent présents en des quantités telles que, quand on met la poudre en solution aqueuse pour l'administrer à un patient,
(i) la concentration d'alpha-1-antitrypsine vaut de 10 à 50 mg/mL,
(ii) la concentration de tréhalose vaut de 10 à 50 mg/mL,
(iii) la concentration de tensioactif vaut de 0,01 à 0,5 % p/v,
(iv) et la concentration de méthionine vaut de 1 à 10 mM.

7. Composition conforme à la revendication 1, qui comprend une alpha-1-antitrypsine (AAT) recombinante et dans laquelle le rapport pondéral AAT/glucide vaut de 1/1 à 5/1.

8. Composition conforme à la revendication 7, dans laquelle le rapport vaut de 1/1 à 2/1.

9. Composition conforme à la revendication 7, dans laquelle le tensioactif est du Polysorbate 80 et l'anti-oxydant est de la méthionine.

10. Composition conforme à l'une des revendications 7 à 9, laquelle composition est formulée en un solide.

11. Composition conforme à l'une des revendications précédentes, dans laquelle l'alpha-1-antitrypsine est une alpha-1-antitrypsine naturelle.

12. Composition conforme à l'une des revendications 1 à 10, dans laquelle l'alpha-1-antitrypsine est une alpha-1-antitrypsine recombinante.

13. Composition conforme à l'une des revendications 1 à 10, dans laquelle l'alpha-1-antitrypsine est un variant d'alpha-1-antitrypsine dont la séquence d'acides aminés est identique, pour au moins 85 %, à la Séquence N° 1 et qui peut inhiber l'élastase.

14. Composition conforme à l'une des revendications 1 à 10, dans laquelle l'alpha-1-antitrypsine est glycosylée.

15. Composition conforme à l'une des revendications 1 à 10, dans laquelle l'alpha-1-antitrypsine est non-glycosylée.

16. Composition, conforme à l'une des revendications 1 à 15, pour utilisation dans un procédé de traitement d'une maladie pulmonaire associée à un déficit d'alpha-1-antitrypsine (AAT), laquelle composition est conçue pour être administrée au niveau des poumons d'un patient.

17. Composition pour utilisation dans le traitement d'une maladie pulmonaire associée à un déficit d'alpha-1-antitrypsine (AAT), conforme à la revendication 16, laquelle composition est conçue pour être administrée par inhalation.

18. Composition pour utilisation dans le traitement d'une maladie pulmonaire associée à un déficit d'alpha-1-antitrypsine (AAT), conforme à la revendication 17, laquelle composition est un solide et est conçue pour être administrée par conversion de ce solide en un aérosol pour inhalation par le patient.

19. Composition pour utilisation dans le traitement d'une maladie pulmonaire associée à un déficit d'alpha-1-antitrypsine (AAT), conforme à l'une des revendications 16 à 18, ladite maladie étant une maladie pulmonaire associée à l'activité d'élastase, de cathepsine G et/ou de protéinase 3.

20. Composition pour utilisation dans le traitement d'une maladie pulmonaire associée à un déficit d'alpha-1-antitrypsine (AAT), conforme à la revendication 19, ladite maladie étant un emphysème.

21. Composition pour utilisation dans le traitement d'une maladie pulmonaire associée à un déficit d'alpha-1-antitrypsine (AAT), conforme à l'une des revendications 16 à 18, ladite maladie étant une maladie pulmonaire associée à l'activation de neutrophiles, de mastocytes ou de lymphocytes T.

22. Composition pour utilisation dans le traitement d'une maladie pulmonaire associée à un déficit d'alpha-1-antitrypsine (AAT), conforme à la revendication 21, ladite maladie étant un asthme.

23. Composition pour utilisation dans le traitement d'une maladie pulmonaire associée à un déficit d'alpha-1-antitrypsine (AAT), conforme à la revendication 21, ladite maladie étant un syndrome de détresse respiratoire de l'adulte, un syndrome de détresse respiratoire du nouveau-né, ou un syndrome septique.

24. Composition pour utilisation dans le traitement d'une maladie pulmonaire associée à un déficit d'alpha-1-antitrypsine (AAT), conforme à l'une des revendications 16 à 18, le patient étant sensible à la maladie et la composition pharmaceutique devant être administrée en une quantité à effet prophylactique.

25. Composition pour utilisation dans le traitement d'une maladie pulmonaire associée à un déficit d'alpha-1-antitrypsine (AAT), conforme à l'une des revendications 16 à 18, le patient étant atteint de la maladie et la composition pharmaceutique devant être administrée en une quantité à effet thérapeutique.
